Europäisches Patentamt

(19) European Patent Office

Office européen des brevets

(11) Numéro de publication : **0 286 506 B1**

(12) **FASCICULE DE BREVET EUROPEEN**

(45) Date de publication du fascicule du brevet :
03.07.91 Bulletin 91/27

(51) Int. Cl.⁵ : **A61L 2/20**

(21) Numéro de dépôt : 88400748.5

(22) Date de dépôt : 28.03.88

(54) **Procédé de stérilisation d'une enceinte étanche et installation pour la mise en oeuvre de ce procédé.**

(30) Priorité : 30.03.87 FR 8704393

(43) Date de publication de la demande :
12.10.88 Bulletin 88/41

(45) Mention de la délivrance du brevet :
03.07.91 Bulletin 91/27

(84) Etats contractants désignés :
AT BE CH DE ES GB IT LI NL SE

(56) Documents cités :
DE-A- 3 523 310
FR-A- 2 335 240
FR-A- 2 352 551
FR-A- 2 354 779
FR-A- 2 508 316

(73) Titulaire : SOCIETE NOUVELLE
D'EXPLOITATION DE LA CALHENE
1 rue du Petit Clamart
F-78140 Velizy Villacoublay (FR)

(72) Inventeur : Picard, Claude
34, rue de Zilina
F-92000 Nanterre (FR)

(74) Mandataire : Mongrédien, André et al
c/o BREVATOME 25, rue de Ponthieu
F-75008 Paris (FR)

## Description

L'invention concerne un procédé de stérilisation d'une enceinte étanche équipée d'un circuit de ventilation et de filtration, ainsi qu'une installation mettant en oeuvre ce procédé.

Dans le brevet FR-A-2 475 679, on a décrit une enceinte étanche équipée d'un circuit de ventilation et de filtration comprenant notamment une conduite d'entrée et une conduite de sortie débouchant dans l'enceinte, des filtres étant placés dans ces conduites à proximité de leurs extrémités débouchant à l'intérieur de l'enceinte. Cette dernière peut être stérilisée au moyen d'un stérilisateur raccordé sur la conduite d'entrée, en amont des filtres. Lorsqu'on désire stériliser l'enceinte, l'agent chimique stérilisant délivré par ce stérilisateur est introduit dans l'enceinte par la mise en oeuvre du circuit de ventilation et de filtration.

Une telle installation a pour avantage de faire passer l'agent stérilisant par les filtres, ce qui garantit une parfaite stérilisation de la totalité du volume interne à l'enceinte.

Cependant, la stérilisation obtenue à l'aide de l'installation décrite dans le document précité est effectuée à l'aide du circuit de ventilation et de filtration, c'est-à-dire de façon dynamique et par un circuit ouvert. Il en résulte que le temps de stérilisation est relativement long et qu'une quantité importante d'agent stérilisant est nécessaire.

De plus, aucun contrôle de l'hygrométrie à l'intérieur de l'enceinte n'est effectué, ce qui conduit à un risque important de dépasser la saturation ou le point de rosée à l'intérieur de l'enceinte. Un tel dépassement du point de rosée est particulièrement défavorable car il conduit à une corrosion importante des parois de l'enceinte et de tous les matériels qu'elle contient ainsi qu'à un risque de dégradation des filtres par déchirement dû au mouillage.

Il est également connu de stériliser une enceinte étanche en introduisant un stérilisateur ou un nébulisateur à l'intérieur de celle-ci. La stérilisation s'effectue alors après avoir fermé les conduites d'entrée et de sortie du circuit de ventilation et de filtration.

Par rapport à la technique décrite précédemment, cette technique présente l'avantage d'assurer une stérilisation plus rapide puisque la stérilisation s'effectue en circuit fermé et de façon statique.

Cependant, cette technique a deux inconvénients. Tout d'abord, l'agent stérilisant ne passe pas par les filtres, de sorte qu'on ne peut pas être sûr que la totalité du volume soit stérile. En outre, aucun contrôle d'hygrométrie n'est effectué, ce qui conduit aux risques décrits précédemment. Dans le cas où l'on utilise un nébulisateur, les effets corrosifs sur les parois et sur les matériels ainsi que la dégradation des filtres sont même pratiquement inévitables, puisque l'on vaporise un brouillard stérilisant à 100% d'hygrométrie.

L'invention a précisément pour objet un procédé de stérilisation d'une enceinte étanche selon lequel on fait circuler l'agent stérilisant au travers des filtres du circuit de ventilation et de filtration pour améliorer la qualité de la stérilisation, on introduit l'agent stérilisant par un circuit fermé permettant une stérilisation statique qui réduit le temps nécessaire à la stérilisation et on contrôle l'hygrométrie afin de ne pas dépasser la saturation ou le point de rosée à l'intérieur de l'enceinte.

Selon l'invention, ce résultat est obtenu au moyen d'un procédé de stérilisation d'une enceinte étanche délimitant un volume clos et équipée d'un circuit de ventilation et de filtration comprenant une conduite d'entrée et une conduite de sortie débouchant dans l'enceinte et pourvues de moyens de filtration, ce procédé étant caractérisé en ce qu'il comprend les étapes successives suivantes :

– fermeture du circuit de ventilation et de filtration en amont des moyens de filtration de la conduite d'entrée et en aval des moyens de filtration de la conduite de sortie,

– séchage du volume clos délimité par l'enceinte jusqu'à l'obtention d'un degré d'hygrométrie inférieur donné,

– introduction dans ce volume d'un agent stérilisant, jusqu'à obtention d'un degré d'hygrométrie supérieur donné, pour lequel le volume est saturé en agent stérilisant, au moyen d'un circuit fermé raccordé sur la conduite d'entrée en amont des moyens de filtration et sur la conduite de sortie en aval des moyens de filtration,

– stérilisation par maintien de l'agent stérilisant dans le volume clos pendant un temps de contact donné, et

– balayage du volume clos par ouverture et mise en oeuvre du circuit de ventilation et de filtration.

Dans un mode de réalisation préféré de l'invention, le séchage du volume clos est réalisé en faisant circuler l'atmosphère interne à ce volume au travers des moyens de dessiccation.

De préférence, pour assurer en permanence une répartition d'ambiance, on effectue un brassage de l'atmosphère interne à l'enceinte, en faisant également circuler cette atmosphère pendant les étapes d'introduction de l'agent stérilisant, de stérilisation et de balayage.

Le séchage de l'atmosphère interne à l'enceinte est réalisé de préférence jusqu'à l'obtention d'un degré d'hygrométrie inférieur voisin de 30%, ce qui permet d'augmenter de façon appréciable la plage d'introduction d'agent stérilisant assurant une meilleure saturation dudit agent. Afin de ne pas dépasser le point de rosée à l'intérieur de l'enceinte, on cesse l'introduction de l'agent stérilisant lorsqu'on atteint un degré d'hygrométrie supérieur voisin de 85%.

Bien que d'autres agents stérilisants tels que le formol puissent être utilisés, on utilise de préférence de l'acide paracétique avec lequel le temps de contact est inférieur à 1 heure.

L'invention concerne également une installation pour la mise en oeuvre du procédé de stérilisation qui vient d'être défini, cette installation étant caractérisée en ce qu'elle comprend :

   – un ensemble de séchage et de brassage apte à être placé à l'intérieur de l'enceinte, cet ensemble comprenant un ventilateur, des moyens de dessiccation et des moyens de filtration montés en série,

   – un circuit d'introduction d'agent stérilisant, apte à être raccordé sur lesdites parties des conduites, respectivement en amont des moyens de filtration de la conduite d'entrée et en aval des moyens de filtration de la conduite de sortie, ce circuit comprenant en série un surpresseur et un évaporateur alimenté en agent stérilisant par l'intermédiaire d'une vanne, ainsi que des vannes d'entrée et de sortie permettant d'isoler le circuit du volume clos pendant la stérilisation, et

   – une sonde de mesure d'hygrométrie, apte à être placée à l'intérieur de l'enceinte, associée à un organe de commande des vannes précitées pour fermer automatiquement ces dernières lorsque l'hygrométrie atteint ledit degré d'hygrométrie supérieur.

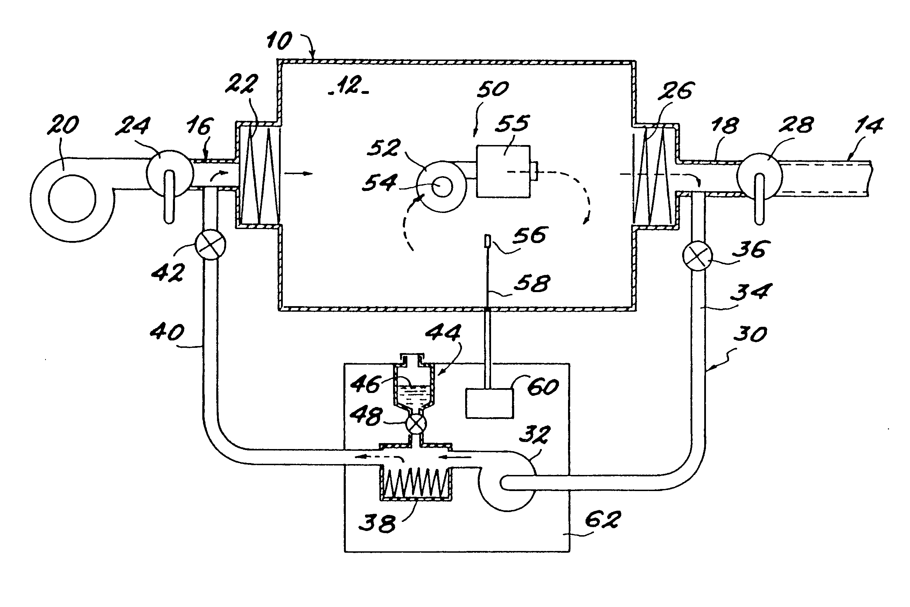

Un mode de réalisation préféré de l'invention va maintenant être décrit, à titre d'exemple nullement limitatif, en se référant au dessin annexé dans lequel la figure unique représente de façon schématique une installation de stérilisation d'une enceinte étanche permettant de mettre en oeuvre le procédé de l'invention.

Sur la figure, on a représenté schématiquement une enceinte étanche 10 délimitant un volume clos 12. L'enceinte 10 peut être utilisée indifféremment en médecine, en pharmacie, en biologie ou en électronique. Les dimensions de l'enceinte sont variables selon son utilisation. A titre d'indication, le volume 12 peut varier entre 2 et 20 m³.

De façon en elle-même classique, l'enceinte 10 est équipée d'un circuit de ventilation et de filtration 14 comprenant une conduite d'entrée 16 et une conduite de sortie 18.

La conduite d'entrée 16 est équipée d'un ventilateur 20 aspirant l'air à l'extérieur pour le refouler dans le volume clos 12 au travers d'un ou plusieurs filtres 22 montés dans la partie de la conduite 16 débouchant à l'intérieur de l'enceinte 10. Une vanne 24 est placée dans la conduite d'entrée 16 en amont du filtre 22.

La conduite de sortie 18 permet à l'air filtré admis à l'intérieur du volume 12 par la conduite 16 d'être rejeté à l'extérieur, afin d'assurer un renouvellement de l'atmosphère régnant à l'intérieur du volume 12. La conduite de sortie 18 est équipée également d'un ou plusieurs filtres 26 dans sa partie adjacente à l'enceinte 32. Une vanne 28 est également placée dans la conduite 18, en aval du filtre 26.

Dans les conditions normales de travail à l'intérieur de l'enceinte 10, les vannes 24 et 28 sont ouvertes et le ventilateur 20 est actionné. Une ventilation de l'atmosphère interne à l'enceinte 10 est ainsi assurée au travers des filtres 22 et 26.

Conformément à l'invention, lorsque le volume de travail 12 à l'intérieur de l'enceinte 10 doit être stérilisé préalablement à son utilisation, on associe à l'enceinte et à son circuit de ventilation et de filtration 14 une installation de stérilisation dont les différents éléments vont maintenant être décrits.

Cette installation comprend tout d'abord un circuit fermé 30 servant à introduire un agent stérilisant à l'intérieur de l'enceinte, après fermeture des vannes 24 et 28. Ce circuit fermé 30 comprend un surpresseur 32 dont l'orifice d'aspiration est raccordé sur une extrémité d'une tuyauterie d'aspiration 34. L'extrémité opposée de cette tuyauterie 34 peut être raccordée sur la conduite de sortie 18 du circuit de ventilation et de filtration 14, entre le filtre 26 et la vanne 28. A proximité de la conduite 18, la tuyauterie 34 est équipée d'une électrovanne 36.

Le circuit 30 comprend de plus un évaporateur à niveau constant 38 dont une tubulure d'entrée communique avec l'orifice de refoulement du surpresseur 32. La tubulure de sortie de l'évaporateur 38 est raccordée sur une première extrémité d'une tuyauterie de refoulement 40. L'extrémité opposée de cette tuyauterie 40 peut être raccordée sur la conduite d'entrée 16 du circuit de ventilation et de filtration, entre la vanne 24 et le filtre 22. A proximité de la conduite 16, la tuyauterie 40 est également équipée d'une électrovanne 42.

Comme l'illustre schématiquement la figure, un réservoir 44 contenant l'agent chimique stérilisant 46 est placé au-dessus de l'évaporateur 38, de façon à alimenter ce dernier par gravité en agent stérilisant 46. Une électrovanne 48 est interposée entre le réservoir 44 et l'évaporateur 38 pour assurer le contrôle et l'interruption de l'alimentation de ce dernier.

En plus du circuit fermé 30 permettant d'introduire l'agent stérilisant à l'intérieur de l'enceinte 10 et des parties attenantes des conduites 16 et 18 comportant les filtres 22 et 26, l'installation de stérilisation selon l'invention comprend un ensemble de séchage et de brassage de l'atmosphère interne au volume clos 12, désigné par la référence 50 sur la figure.

Cet ensemble 50, qui est prévu pour être placé directement à l'intérieur du volume clos 12, comprend un ventilateur 52 aspirant directement l'air à l'intérieur du volume 12. L'air aspiré par le ventilateur 52 est refoulé à l'intérieur du volume clos 12 au travers d'une cartouche dessiccatrice 54, puis d'un filtre 55. Comme on le verra plus en détail ultérieurement, cet ensemble 50 a pour double fonction d'assurer le séchage du volume 12 avant la mise en oeuvre du circuit 30 et de permettre un brassage permanent de l'air contenu dans ce volume tout au long des différentes étapes de la stérilisation.

Enfin, l'installation de stérilisation selon l'invention comprend une sonde 56 placée à l'intérieur de l'enceinte 10 afin de mesurer en permanence l'hygrométrie à l'intérieur de cette enceinte. Cette sonde 56 est reliée par un conducteur électrique 58 à un circuit électronique 60 placé à l'extérieur de l'enceinte et servant à commander automatiquement la fermeture des électrovannes 36, 42 et 48 lorsque l'hygrométrie à l'intérieur de l'enceinte atteint un seuil maximum donné.

Dans la pratique et comme on l'a représenté schématiquement sur la figure, le circuit électronique 60, la pompe 32, l'évaporateur 38, le réservoir 44 et la vanne 48 sont montés à l'intérieur d'un boîtier unique pour former un appareil 62.

Conformément à l'invention, lorsqu'on désire stériliser une enceinte 10 à l'aide de l'appareil décrit précédemment, on raccorde les tuyauteries 34 et 40 respectivement sur les conduites de sortie 18 et d'entrée 16 du circuit de ventilation et de filtration 14 de l'enceinte ; à cet effet, ces conduites sont équipées de dérivations normalement obturées par des bouchons (non représentés).

On ferme ensuite les vannes 24 et 28 du circuit 14 afin d'isoler un volume clos comprenant le volume 12 interne à l'enceinte 10 ainsi que les parties des extrémités des conduites 16 et 18 attenantes à cette enceinte et dans lesquelles sont montés les filtres 22 et 26.

Par ailleurs, on met en place la sonde hygrométrique 56 ainsi que l'ensemble 50 à l'intérieur de l'enceinte étanche 10. Enfin, le réservoir 44 est rempli d'agent stérilisant 46 et un élément neuf est introduit dans l'évaporateur 38 à niveau constant.

Lorsque ces préparatifs sont terminés, le procédé selon l'invention est mis en oeuvre sous la forme d'une première étape consistant à assécher le volume 12 interne à l'enceinte étanche 10. A cet effet, les vannes 36 et 42 étant fermées, on actionne le ventilateur 52 de façon à faire circuler l'air contenu dans le volume 12 sur la cartouche dessiccatrice 54 et au travers du filtre 55. Cette phase de séchage est poursuivie jusqu'à ce que le taux d'hygrométrie mesuré par la sonde 56 à l'intérieur du volume 12 atteigne une valeur inférieure généralement voisine de 30%. Le circuit électronique 60 associé à la sonde indique à l'opérateur que cette valeur est atteinte, par exemple en affichant un signal approprié.

Il est à noter que la durée de cette première étape dépend essentiellement de l'hygrométrie qui régnait initialement à l'intérieur de l'enceinte et du volume interne à celle-ci. Toutefois, ce temps reste toujours relativement court étant donné que l'air confiné à l'intérieur de l'enceinte circule en circuit fermé sur la cartouche dessicatrice 54.

Lorsque cette première étape est terminée, le circuit 30 est mis en oeuvre afin d'introduire dans le volume 12 interne à l'enceinte 10 l'agent stérilisant 46 contenu dans le réservoir 44. Cet agent stérilisant est de préférence de l'acide paracétique, mais il peut aussi être constitué de tout autre agent chimique stérilisant tel que du formol.

Pour réaliser cette introduction, les vannes 36 et 42 sont ouvertes, de même que la vanne 48 qui alimente en agent stérilisant 46 l'évaporateur 38 en maintenant constant le niveau de l'agent stérilisant dans cet évaporateur. Simultanément, le surpresseur 32 est actionné, ce qui a pour effet de faire circuler l'air contenu dans le volume 12 interne à l'enceinte 10 dans l'évaporateur 38, par l'intermédiaire du circuit 30. Etant donné que cet air a été préalablement asséché à l'aide de l'ensemble 50, on introduit ainsi en un temps relativement court une quantité importante de vapeur d'agent stérilisant à l'intérieur de l'enceinte.

Il est à noter que le débit de l'air à l'intérieur du circuit 30 créé par le surpresseur 32 est relativement important afin d'engendrer, notamment dans les filtres 22 et 26, une certaine pression tendant à évaporer les particules d'agent stérilisant liquide qui tendent à se fixer sur ces filtres. De plus, les sections des tuyauteries 34 et 40 sont inférieures à celles des conduites 16 et 18 afin que cet effet puisse être obtenu quelles que soient les dimensions de l'enceinte 10 à stériliser.

Tout au long du fonctionnement du circuit 30 permettant d'introduire l'agent stérilisant à l'intérieur du volume 12 délimité par l'enceinte 10, le ventilateur 52 continue à fonctionner afin d'homogénéiser en permanence l'air contenu dans l'enceinte. De cette manière, la concentration en agent stérilisant est pratiquement uniforme à tout instant, en tout point de l'enceinte.

Lorsque la sonde 56 détecte à l'intérieur de l'enceinte 10 un degré d'hygrométrie correspondant à la saturation de l'air en agent stérilisant, le circuit électronique 60 commande automatiquement l'arrêt du surpresseur 32 et la fermeture des vannes 36, 42 et 48. Dans la pratique, cette saturation est atteinte lorsque le degré d'hygrométrie à l'intérieur de l'enceinte 10 est voisin de 85% (±5%). En limitant ainsi l'introduction de l'agent stérilisant à une valeur correspondant au point de rosée à l'intérieur du volume 12, on évite les inconvénients liés au dépassement de ce point de rosée tels que la corrosion de l'enceinte et des appareils qu'elle contient et que le risque de déchirement des filtres.

Par ailleurs, étant donné que l'introduction de l'agent stérilisant s'effectue en circuit fermé, le temps nécessaire pour atteindre la saturation est relativement court.

Le procédé selon l'invention se poursuit ensuite par une étape correspondant à la stérilisation proprement dite, au cours de laquelle l'agent stérilisant emprisonné à l'intérieur du volume 12 est maintenu en contact avec l'atmosphère interne à l'enceinte pendant un temps qui est fonction de la nature de l'agent stérilisant utilisé. Ainsi, dans le cas où cet agent stérilisant est de l'acide paracétique, la durée de cette phase de contact est inférieure à 1 heure (par exemple environ 40 mn) alors que cette même phase de contact atteint environ 12 heures lorsque l'agent stérilisant utilisé est du formol.

Au cours de cette phase de contact assurant la stérilisation proprement dite, le ventilateur 52 continue à fonctionner, toujours dans un souci d'homogénéité de l'air contenu dans le volume 12.

A la fin de cette phase de contact, l'agent stérilisant est évacué par un balayage du volume 12. Ce balayage est réalisé en ouvrant les vannes 24 et 28 du circuit de ventilation et de filtration 14, puis en actionnant le ventilateur 20. Simultanément, le ventilateur 52 de l'ensemble 50 continue à fonctionner.

Afin de rincer le circuit de stérilisation 30, le surpresseur 32 peut être remis en route pendant environ un quart d'heure après ouverture des vannes 36 et 42, la vanne d'alimentation 48 en agent stérilisant étant maintenue fermée. Cette opération peut être effectuée soit pendant la phase de balayage décrite précédemment, soit éventuellement après cette opération.

Lorsque le balayage du volume 12 interne à l'enceinte 10 a été effectué, de même que le rinçage du circuit 30, les tuyauteries 34 et 40 peuvent être déconnectées des conduites 18 et 16. L'ensemble 50 ainsi que la sonde 56 peuvent aussi être sortis de l'enceinte 10. Cette dernière peut alors être utilisée pour y effectuer les opérations souhaitées en atmosphère stérile.

L'introduction et l'extraction de l'ensemble 50 et de la sonde 56 dans l'enceinte 10 et hors de celle-ci sont réalisées au moyen de dispositifs de transfert étanche de type connu qui ne font pas partie de la présente invention.

**Revendications**

1. Procédé de stérilisation d'une enceinte étanche (10) délimitant un volume clos (12) et équipée d'un circuit de ventilation et de filtration (14) comprenant une conduite d'entrée (16) et une conduite de sortie (18) débouchant dans l'enceinte (10) et pourvues de moyens de filtration (22, 26), ce procédé étant caractérisé en ce qu'il comprend les étapes successives suivantes :
    – fermeture du circuit de ventilation et de filtration (14) en amont des moyens de filtration (22) de la conduite d'entrée (16) et en aval des moyens de filtration (26) de la conduite de sortie (18),
    – séchage du volume clos (12) délimité par l'enceinte (10) jusqu'à l'obtention d'un degré d'hygrométrie inférieur donné,
    – introduction dans ce volume d'un agent stérilisant (46) jusqu'à obtention d'un degré d'hygrométrie supérieur donné, pour lequel le volume (12) est saturé en agent stérilisant, au moyen d'un circuit fermé (30) raccordé sur la conduite d'entrée (16) en amont des moyens de filtration (22) et sur la conduite de sortie (18) en aval des moyens de filtration (26),
    – stérilisation par maintien de l'agent stérilisant (46) dans le volume clos (12) pendant un temps de contact donné, et
    – balayage du volume clos par ouverture et mise en oeuvre du circuit de ventilation et de filtration (14).

2. Procédé de stérilisation selon la revendication 1, caractérisé en ce que le séchage du volume clos (12) est réalisé en faisant circuler l'atmosphère interne à ce volume au travers de moyens de dessiccation (54).

3. Procédé selon la revendication 2, caractérisé en ce qu'on fait également circuler l'atmosphère interne audit volume clos (12) pendant le étapes d'introduction de l'agent stérilisant, de stérilisation et de balayage.

4. Procédé selon l'une quelconque des revendications 1 à 3, caractérisé en ce que le degré d'hygrométrie inférieur est voisin de 30%.

5. Procédé selon l'une quelconque des revendications 1 à 4, caractérisé en ce que le degré d'hygrométrie supérieur est voisin de 85%.

6. Procédé selon l'une quelconque des revendications 1 à 5, caractérisé en ce qu'on utilise de l'acide paracétique comme agent stérilisant, le temps de contact étant inférieur à 1 heure.

7. Installation pour la mise en oeuvre du procédé de stérilisation selon l'une quelconque des revendications 1 à 6, caractérisée en ce qu'elle comprend :
    – un ensemble de séchage et de brassage (50) apte à être placé à l'intérieur de l'enceinte (10), cet ensemble comprenant un ventilateur (52), des moyens de dessiccation (54) et des moyens de filtration (55) montés en série,
    – un circuit d'introduction (30) d'agent stérilisant (46), apte à être raccordé sur lesdites conduites (16, 18), respectivement en amont des moyens de filtration (22) de la conduite d'entrée (16) et en aval des moyens de filtration (26) de la conduite de sortie (18), ce circuit comprenant en série un surpresseur (32) et un évaporateur (38) alimenté en agent stérilisant (46) par l'intermédiaire d'une vanne (24), ainsi que des vannes d'entrée et de sortie (28) permettant d'isoler le circuit du volume clos (12) pendant la stérilisation, et
    – une sonde (56) de mesure d'hygrométrie, apte à être placée à l'intérieur de l'enceinte (10), associée à un organe (60) de commande des vannes précitées pour fermer automatiquement ces dernières lorsque l'hygrométrie atteint ledit degré d'hygrométrie supérieur.

## Claims

1. Process for the sterilization of a tight enclosure defining a closed volume (12) and equipped with a ventilation and filtration circuit (14) having a supply pipe (16) and a discharge pipe (18) issuing into the enclosure (10) and provided with filtration means (22, 26), characterized in that it comprises the following successive stages :
- closing the ventilation and filtration circuit (14) upstream of the filtration means (22) of the supply pipe (16) and downstream of the filtration means (26) of the discharge pipe (18),
- drying the closed volume (12) defined by the enclosure (10) until a given lower relative humidity is obtained,
- introduction of a sterilizing agent (46) into said volume (12) until a given higher relative humidity is obtained and for which the volume is saturated with sterilizing agent, by using a closed circuit (30) connected to the supply pipe (16) upstream of the filtration means (22) and to the discharge pipe (18) downstream of the filtration means (26),
- sterilization by maintaining the sterilizing agent (46) in the closed volume (12) for a given contact time and
- scavenging the closed volume by opening and using the ventilation and filtration circuit (14).

2. Sterilization process according to claim 1, characterized in that the drying of the closed volume (12) is carried out by circulating the atmosphere within said volume through drying means (54).

3. Process according to claim 2, characterized in that there is also a circulation of the atmosphere within the closed volume (12) during the sterilizing agent introduction, sterilization and scavenging stages.

4. Process according to any one of the claims 1 to 3, characterized in that the lower relative humidity level is close to 30%.

5. Process according to any one of the claims 1 to 4, characterized in that the upper relative humidity level is close to 85%.

6. Process according to any one of the claims 1 to 5, characterized in that p-acetic acid is used as the sterilizing agent, the contact time being less than 1 hour.

7. Installation for performing the sterilization process according to any one of the claims 1 to 6, characterized in that it comprises :
- a drying and stirring assembly (50) which can be placed within the enclosure (10), said assembly incorporating a fan (52), drying means (54) and filtration means (55) connected in series,

- a sterilizing agent (46) introduction circuit (30), which can be connected to said parts of the pipes (16, 18), respectively upstream of the filtration means (22) of the supply pipe (16) and downstream of the filtration means (26) of the discharge pipe (18), said circuit having in series a blower (32) and an evaporator (38) supplied with sterilizing agent (46) via a valve (24), as well as inlet and outlet valves (28) making it possible to isolate the circuit from the closed volume (12) during sterilization and
- a relative humidity measuring probe (56), which can be placed within the enclosure (10) and associate-d with a control member (16) for the aforementioned valves in order to automatically close the latter when the relative humidity reaches said upper relative humidity level.

## Ansprüche

1. Verfahren zum Sterilisieren eines dichten Behälters (10), der ein geschlossenes Volumen (12) begrenzt und mit einem Ventilations- und Filtrationskreislauf (14) ausgestattet ist und der ein Eingangsrohr (16) und ein Ausgangsrohr (18) umfaßt, die in den Behälter (10) münden und mit Filtervorrichtungen versehen sind, wobei dieses Verfahren dadurch gekennzeichnet ist, daß es folgende, sukzessive Verfahrensschritte aufweist :
- schließen des Ventilations- und Filtrationskreislaufs (14) oberhalb der Filtervorrichtungen (22) des Eingangsrohres und unterhalb der Filtervorrichtungen (26) des Ausgangsrohres (18),
- Trocknen des von dem Behälter (10) begrenzten, geschlossenen Volumens (12) bis zum Erhalt eines gegebenen, niedrigeren Hygrometriewertes,
- Einführen eines Sterilisationsmittels (46) in dieses Volumen bis zum Erhalt eines gegebenen, höheren Hygrometriewertes, bei dem das Volumen (12) mit dem Sterilisationsmittel gesättigt ist, mittels eines geschlossenen, an dem Eingangsrohr (16) oberhalb der Filtervorrichtungen (22) und an dem Ausgangsrohr (18) unterhalb der Filtervorrichtungen (26) angeschlossenen Kreislaufs (30),
- Sterilisation durch Aufrechterhalten des Sterilisationsmittels (46) in dem geschlossenen Volumen (12) während einer vorgegebenen Kontaktzeit, und
- Säubern des geschlossenen Volumens durch Öffnen und Inbetriebnahme des Ventilations- und Filtrationskreislaufs (14).

2. Sterilisationsverfahren nach Anspruch 1, dadurch gekennzeichnet, daß das Trocknen des geschlossenen Volumens (12) durchgeführt wird, indem man die innere Atmosphäre dieses geschlossenen Volumens durch Trockenvorrichtungen (54) zirkulieren läßt.

3. Verfahren nach Anspruch 2, dadurch gekennzeichnet, daß man die innere Atmosphäre dieses geschlossenen Volumens (12) ebenfalls während den Schritten der Einführung des Sterilisationsmittels, der Sterilisation und des Säuberns zirkulieren läßt.

4. Verfahren nach einem der Ansprüche 1 bis 3, dadurch gekennzeichnet, daß der niedrigere Hygrometriewert in der Nähe von 30% liegt.

5. Verfahren nach Anspruch 1 bis 4, dadurch gekennzeichnet, daß der höhere Hygrometriewert in der Nähe von 85% liegt.

6. Verfahren nach einem der Ansprüche 1 bis 5, dadurch gekennzeichnet, daß man Parazetsäure als Sterilisationsmittel verwendet, wobei die Kontaktzeit kleiner als 1 Stunde ist.

7. Vorrichtung zum Durchführen des Sterilisationsverfahrens nach einem der Ansprüche 1 bis 6, dadurch gekennzeichnet, daß sie umfaßt :

– eine Trocken- und Umwälzvorrichtung (50), die geeignet ist, im Innern des Behälters (10) angeordnet zu werden, wobei diese Vorrichtung in Reihe montiert einen Ventilator (52), Trockenvorrichtungen (54) und Filtervorrichtungen (55) umfaßt,

– einen Einführungskreislauf (30) für das Sterilisationsmittel (46), der geeignet ist mit den Rohren (16, 18) jeweils oberhalb der Filtervorrichtungen (22) des Eingangsrohres (16) und unterhalb der Filtervorrichtungen (26) des Ausgangsrohres (18) verbunden zu werden, wobei dieser Kreislauf in Reihe eine Pumpe (32) und einen Verdampfer (38), der mit dem Sterilisationsmittel (46) über ein Ventil (24) versorgt wird, ebenso wie Eingangs- und Ausgangsventile (28) umfaßt, die ermöglichen, den Kreislauf des geschlossenen Volumens (12) während der Sterilisation zu isolieren, und

– eine Sonde (56) zum Messen der Hygrometrie, die geeignet ist, innerhalb des Behälters (10) angeordnet zu werden und die mit einem Steuerungselement (60) für die obengenannten Ventile verbunden ist, um automatisch letztere zu schließen, sobald der Hygrometriewert den höheren Hygrometriewert erreicht.